# EUROPEAN PATENT APPLICATION

(11) **EP 2 255 807 A1**
(43) Date of publication of application: **01.12.2010**
(21) Application number: 09290383.0
(22) Date of filing: 26.05.2009
(51) Int. Cl.: A61K 31/437, A61K 31/15, A61P 25/20

(54) **Method of treating sleep disorders using the combination of eplivanserin and zolpidem**

(71) Applicant: Sanofi-Aventis, 75013 Paris (FR)
(72) Inventor: The designation of the inventor has not yet been filed
(74) Representative: Morel-Pécheux, Muriel

(57) **Abstract**

The instant invention relates to a method of treating sleep disorders by using eplivanserin or pharmaceutically acceptable salts or esters thereof and zolpidem by evaluating the effect of eplivanserin combined with the sleep-inducing agent zolpidem on psychomotor/cognitive performance.

## Description

The instant invention relates to a method of treating sleep disorders by using eplivanserin or pharmaceutically acceptable salts or esters thereof and zolpidem by evaluating the effect of eplivanserin combined with the sleep-inducing agent zolpidem on psychomotor/cognitive performance.

Nocturnal awakenings and difficulty resuming sleep are common symptoms of insomnia. Compared to classical insomnia symptoms, they are associated with higher rates of daytime cognitive impairment. It appears that reduced EEG slow-wave activity during slow-wave sleep (SWS) is directly correlated with an increase in nocturnal awakenings.

The serotonin type two (5-HT₂) receptors influence nocturnal awakenings via tonic inhibition of slow-wave sleep (SWS). Antagonism of 5-HT_{2A} receptors has been shown to increase SWS and enhance slow-wave activity in humans.

The compound (1Z, 2E)-1-(2-fluorophenyl)-3-(4-hydroxyphenyl)-prop-2-en-1-one-O-(2-dimethylaminoethyl)oxime, is hereafter referenced as eplivanserin or pharmaceutically acceptable salts or esters thereof. Documents EP 373998 and US 5166416 claim compounds having a generic scope encompassing eplivanserin or pharmaceutically acceptable salts or esters thereof and also specifically claimed eplivanserin or pharmaceutically acceptable salts or esters thereof, and disclosed its use as a platelet anti-aggregant or a psychotropic agent.

See also US 6143792 and EP 1014960 (sleep apnea) and US 6576970 and EP 11140955 (snoring and upper airway resistance syndrome).

Eplivanserin or pharmaceutically acceptable salts or esters thereof, in particular hemifumarate salt, is an antagonist of 5HT_{2A} receptors (Journal of Pharmacological Experiment in Therapeutics, (1992), vol. 262 (2), pp. 759-68). Eplivanserin is well absorbed (>70%). Conventional dosage, between 1 and 10 mg, leads to a maximal plasma concentration that is reached between 2 and 6 hours; the half-life time of eplivanserin or pharmaceutically acceptable salts or esters thereof is relatively long, with an average value of 50 hours. Eplivanserin or pharmaceutically acceptable salts or esters thereof is also known to enhance slow wave sleep (SWS) (Neuropsychopharmacology (1999), vol.21 (3), pp. 455-466).
It has been recently discovered that eplivanserin or pharmaceutically acceptable salts or esters thereof, by enhancing SWS, may significantly improve sleep maintenance and quality of sleep (QoS) without inducing next-day sedation and rebound of insomnia or withdrawal symptoms after treatment discontinuation in patients with chronic insomnia and sleep maintenance difficulties.
Eplivanserin is suitable for the preparation of a sleep pharmacotherapy at a dose of 5 mg once daily at dinner time or in the evening. Unlike benzodiazepines, eplivanserin does not bind to GABA receptors.

Zolpidem is a short-acting hypnotic agent, which acts as a GABA-A receptor modulator. Zolpidem belongs to the class of imidazopyridines, and is administered orally in the form of an immediate-release tablet or in a galenical form allowing delayed release.

Eplivanserin will be used in patients suffering from sleep disorders, and, in particular in patients suffering from insomnia characterized by difficulties with sleep maintenance. However, these patients may also complain occasionally of sleep induction difficulties. It is found now that the psychomotor and cognitive performances of subjects treated with eplivanserin combined with the sleep-inducing agent zolpidem are not impaired as compared to a single administration of zolpidem alone.

Thus, one embodiment of the invention relates to the use of eplivanserin or pharmaceutically acceptable salts or esters thereof in combination with zolpidem for the preparation of a medication for the treatment of sleep disorders in a patient without impairing its psychomotor and cognitive performances, as compared to zolpidem alone.

In some embodiments, sleep disorders are insomnia.
In some embodiments, sleep disorders are chronic insomnia.
In some embodiments, sleep disorders are insomnia characterized by sleep maintenance difficulties.
In some embodiments, sleep disorders are insomnia including nocturnal awakenings, usually for short-term duration.
In some embodiments, sleep disorders are insomnia including nocturnal awakenings or early awakenings, usually for short-term duration.
In some embodiments, sleep disorders are chronic insomnia characterized by difficulties with sleep maintenance.
In some embodiments, sleep disorders are insomnia characterized by sleep induction difficulties and sleep maintenance difficulties.
In some embodiments, sleep disorders are insomnia characterized by difficulties in falling asleep and sleep maintenance difficulties.

In some embodiments, sleep disorders are insomnia characterized by difficulties in falling asleep and nocturnal awakenings

The next-day psychomotor and cognitive performances after repeated administration of eplivanserin 5 mg were measured and the hypnotic drug flurazepam at a dose of 30 mg was the active control.

### Example 1: Study design

Single center study employing a two-part, double-blind, randomized, placebo- and active-controlled, 4-way crossover design (Figure 1). Eligibility criteria included certification of good health, 18-50 years of age, and written informed consent.
Part A (the first part of the study): participants were first administered either one evening dose of flurazepam 30 mg or matching placebo, and after a 21-day washout period, participants were administered the other treatment.
Part B: participants received either an evening dose of eplivanserin 5 mg or matching placebo for 21 days, followed by co-administration with a single dose of zolpidem-IR 10 mg. After this phase of the study, participants were then administered the reverse treatment sequence.
Next-day morning (7:05-7:25 am), cognitive and psychomotor performance was assessed using 6 serial tests:
■ Bond-Lader Visual Analog Scale (VAS)
■ Immediate recall of Word list
■ Compensatory Tracking Task (CTT)
■ Critical Flicker Fusion (CFF) test
■ Choice Reaction Time (CRT)
■ Delayed Recall of Word Lists

The figure 1 shows the process relative to the conducted study.

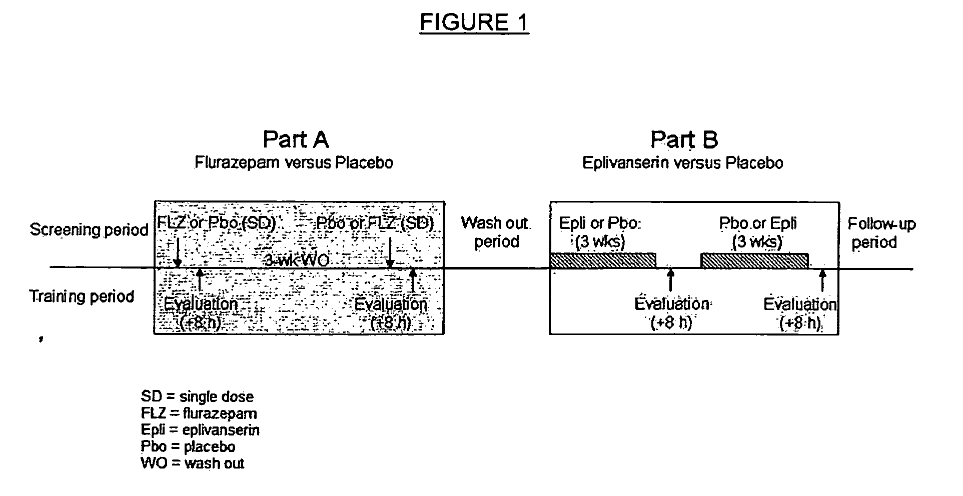

Seven primary endpoints were derived from the 6 following psychometric tests:
■ Bond and Lader Visual Analogue Scale (VAS), showing the alertness ;
■ Immediate recall of Word List, showing the number of correct words ;
■ Compensatory Tracking test (CTT) , showing both the mean deviation and the mean response time ;
■ Critical Flicker Fusion (CFF) threshold;
■ Choice Reaction Time (CRT) , showing the total reaction time
■ Delayed recall of Word List, showing the number of correct words.

For parts A and B, these 7 primary endpoints (raw data) were first analyzed simultaneously using a multivariate mixed effect model (global analysis). Then for each primary endpoint (individual analysis), the difference compared with placebo was derived from the model.
For each study part, percent change from placebo was computed for each participant and each primary endpoint.

### Example 2: Statistical analysis

Adverse events were recorded (Medical Dictionary for Regulatory Activities v9.1). Other measures included vital signs, standard clinical laboratory parameters (biochemistry, hematology, and urinalysis), and electrocardiograms.

Twenty-four healthy individuals were recruited and completed the study: 15 males (62.5%) and 9 females (37.5%), with a median age of 28.2 years (range, 19-49).

### Pharmacokinetics:

Steady-state levels were reached for both eplivanserin and its active metabolite, after 20 days of repeated administration of eplivanserin 5 mg. As a consequence, cognitive/psychomotor performance was assessed under steady state conditions.

### Pharmacodynamics

### Example 2.1: Part A: flurazepam versus placebo

Flurazepam was associated with an overall treatment effect on the 7 primary endpoints compared with placebo (global analysis, P=0.0001).
Individual analysis of each primary endpoint showed statistically significant differences between placebo and flurazepam in 6 of the 7 parameters: total reaction time (P=0.0003); immediate word recall - number of correct responses (P=0.0001); delayed word recall - number of correct responses (P=0.0001); CTT - overall average tracking error (P=0.0221); CTT - overall average response time (P=0.0029); Bond and Lader VAS - alertness (P=0.0031) (Table 1).
CFF threshold did not reach statistical significance (P=0.2005).

**Table 1: Cognitive and psychomotor performances following administration of flurazepam (percent change from placebo).**

| **Test** | **Parameter** | **Mean difference estimate** |
|---|---|---|
| CFF | Point of subjective equality (Hz) | -0.52 |
| CRT | Total reaction time (ms) | 47.46 |
| Immediate word recall | Number of correct responses | -3.08 |
| Delayed word recall | Number of correct responses | -4.38 |
| Compensatory tracking task | Overall average tracking errors (pixels) | 1.64 |
| | Overall average response time (ms) | 49.98 |
| Bond and Lader visual analogue scale | Alertness | 62.38 |

### Example 2.2: Part B: eplivanserin versus placebo

Eplivanserin alone was not associated with an overall treatment effect on the 7 primary endpoints compared with placebo (global analysis, P=0.0603). Individual analysis of each primary endpoint revealed non-statistically significant differences compared with placebo (P>0.1), with the exception of CFF reaching statistical significance (P=0.0002) (Table 2).

**Table 2: Cognitive and psychomotor performances following administration of eplivanserin (percent change from placebo)**

| **Test** | **Parameter** | **Mean difference estimate** |
|---|---|---|
| CFF | Point of subjective equality (Hz) | -1.01 |
| CRT | Total reaction time (ms) | -10.40 |
| Immediate word recall | Number of correct responses | -1.04 |
| Delayed word recall | Number of correct responses | -0.33 |
| Compensatory tracking task | Overall average tracking errors (pixels) | -0.22 |
| | Overall average response time (ms) | 13.03 |
| Bond and lader visual analogue scale | alertness | 9.38 |

### Example 2.3: Part B: eplivanserin plus zolpidem versus placebo plus zolpidem

Eplivanserin plus zolpidem was not associated with an overall treatment effect on the 7 primary endpoints compared with placebo plus zolpidem (global analysis, P=0.1337). Individual analysis of each primary endpoint revealed non-statistically significant differences compared with placebo + zolpidem (P>0.0533), with the exception of CFF reaching statistical significance (P=0.002) (Table 3).

**Table 3: The general absence of effect of eplivanserin + zolpidem compared with placebo + zolpidem on next-day cognitive/psychomotor performance.**

| **Test** | **Parameter** | **Mean difference estimate** |
|---|---|---|
| CFF | Point of subjective equality (Hz) | -1.07 |
| CRT | Total reaction time (ms) | 4.83 |
| Immediate word recall | Number of correct response | -1.04 |
| Delayed word recall | Number of correct responses | -0.93 |
| Compensatory tracking task | Overall average tracking errors (pixels) | -1.07 |
| | Overall average ersponse time (ms) | 0.5 |
| Bond and ladder visual analogue scale | alertness | 16.82 |

### Safety

There were no treatment discontinuations due to adverse events.
In Part A (flurazepam vs placebo), few adverse events (2/24 participants in each group) were reported.
In Part B (eplivanserin vs placebo), the most frequently reported adverse events were: headache in 25% (6/24) of participants in both the placebo and eplivanserin groups; and dizziness, somnolence and asthenia reported by 29.2% (7/24) in the eplivanserin group versus 4.2% (1/24) in the placebo group.
In Part B (eplivanserin plus zolpidem vs placebo plus zolpidem), few adverse events (1/24 participants in each group) were reported.

The previous examples show that repeated evening administration of eplivanserin 5 mg to healthy individuals did not produce significant effects on next-day psychomotor and cognitive performance compared with placebo. This was in contrast with single-dose administration of flurazepam 30 mg (positive control). Flurazepam 30 mg had significant effects on next-day cognitive and psychomotor performance compared with placebo.

When co-administered with zolpidem, there was no significant effect on next-day performance when compared with zolpidem alone.
A decrease in CFF threshold was observed after administration of eplivanserin. This is likely to be caused by pupillary constriction, an acknowledged effect of other 5-HT2 antagonists, which in turn affects CFF thresholds.
Eplivanserin 5 mg was well tolerated alone and in combination with zolpidem.

By enhancing restorative SWS, eplivanserin reduces nocturnal awakenings in patients with sleep maintenance insomnia without, impairing the next-day functioning. Results of this study in healthy subjects suggest that in patients suffering from sleep disorders, eplivanserin in association with zolpidem will not impair next-day cognitive and psychomotor performances, as compared to zolpidem alone.

## Claims

1. Eplivanserin or pharmaceutically acceptable salts or esters thereof in combination with zolpidem for the preparation of a medicament for the treatment of sleep disorders in a patient without impairing its psychomotor and cognitive performances.

2. Eplivanserin according to claim 1, wherein eplivanserin salt is hemifumarate.

3. Eplivanserin according to claim 1 or 2, wherein sleep disorders are insomnia.

4. Eplivanserin according to claim 1 to 3, wherein sleep disorders are chronic insomnia.

5. Eplivanserin according to claim 1 to 4, wherein sleep disorders are insomnia **characterized by** difficulties with sleep maintenance.

6. Eplivanserin according to claims 1 to 5, wherein sleep disorders are chronic insomnia **characterized by** difficulties with sleep maintenance.

7. Eplivanserin according to claims 1 or 2 wherein sleep disorders are insomnia **characterized by** sleep induction difficulties or difficulties in falling asleep associated with difficulties with sleep maintenance or nocturnal awakenings.

8. Eplivanserin according to claims 1 to 7, wherein a therapeutic amount of eplivanserin is 5 mg once a day.

9. Eplivanserin according to claims 1 to 8, wherein the combination is simultaneous, separate or sequential over time.
